# EUROPEAN PATENT APPLICATION

(11) **EP 3 184 047 A1**
(43) Date of publication of application: **28.06.2017**
(21) Application number: 15202478.2
(22) Date of filing: 23.12.2015
(51) Int. Cl.: A61B 6/00, A61N 5/10

(54) **PHANTOM FOR RADIATION DOSIMETRY**

(71) Applicant: ION BEAM APPLICATIONS S.A., 1348 Louvain-la-Neuve (BE)
(72) Inventor: MENICHELLI, David, 92353 Postbauer-Heng (DE)
(74) Representative: Pecher, Nicolas

(57) **Abstract**

Phantom for radiation dosimetry comprising a main body of material, a receptacle having the shape of a developable surface, a radiation sensitive film located in the said receptacle, the main body of material comprises one or more radiation detectors each for measuring a dose received at a location of said radiation sensitive film. The invention also relates to the method for calibrating a radiation sensitive film used in the phantom according to the invention.

## Description

### Field of the invention

According to a first aspect, the invention relates to phantom for radiation dosimetry and more precisely to a phantom comprising a radiation sensitive film and a method of film calibration thereof.

### Description of prior art

One knows phantoms for radiation dosimetry that are used for pre-treatment plan verification, for machine quality assurance or for linear accelerator (LINAC) commissioning. For example, in medical applications, a radiation treatment is planned to deliver the appropriate beam radiotherapy treatment to a patient. This plan can be tested during the pre-treatment or online plan verification. Machine quality assurance may be performed on a regular basis, e.g. daily. LINAC commissioning is usually performed at delivery of a new machine. In all cases, the dose received by a patient or by an object must be measured with radiation detectors. These detectors are, at least sometimes, radiation sensitive films. These films have a high spatial resolution and high dosimetric performances, such as dose rate linearity and sensitivity independence on beam quality. However, sensitive films are not intensively used because their calibration procedure is complex and time consuming.

There exist two kinds of sensitive films: radiographic and radiochromic. Radiographic films need a development while radiochromic ones are self-developing: they spontaneously change colour after irradiation. Dosimetric performances of radiochromic film are higher, because they are organic film and do not contains elements with high atomic number.

For example, radiochromic films are calibrated as a batch. One film of the batch is irradiated with a 2D distribution including spots of different and known dose (usually 8-16). After the scan, each spot generates a calibration point, which can be interpolated to obtain the calibration curve. However, since the opacity of the film significantly increases during the first hours after irradiation, one has to wait 24h before scanning a film, to be sure that the actual film and the one used for calibration are used in the same conditions. This waiting time largely affects the efficiency of dosimetric procedures based on films. In addition to low efficiency, the accuracy of this method is limited by variations with time of the scanner and of the stored films.

A faster procedure is described by Lewis et al., in Med. Phys. 39 (2012) 6339 and in US20150041632. This procedure consists to calibrate independently each film, based on just one calibration point. In this case the film to be calibrated, and two small film strips (one un-irradiated and the other irradiated to the highest dose of the treatment) are scanned at the same time. As the strip is irradiated just after the film, the scan can be performed already after 20-30min. However, this procedure relies on the assumption that the form of the calibration curve is known which is not always the case.

Moreover, in modern radiotherapy treatments, dose distribution may have steep gradients due to the use of multi-leaf collimators and/or treatment units with many degrees of freedom and a large dose may be delivered in a single irradiation fraction (e.g. stereotactic treatments). Therefore, there is a need for an accurate dose measurement with high spatial accuracy.

### Summary of the invention

The present invention is defined in the appended independent claims. Preferred embodiments are defined in the dependent claims.

According to a first aspect, the invention relates to a phantom for radiation dosimetry. Preferably, this phantom comprises a main body of material that comprises a receptacle having the shape of a developable surface, and a radiation sensitive film located in the said receptacle. This phantom is characterised in that, said main body of material further comprises one or more radiation detectors each for respectively measuring a dose of radiation which reached a specific location of said radiation sensitive film.

Preferably, the phantom is designed to be used in the radiotherapy context in which the radiation beam are usually photons or electrons beams, neutrons, protons, or pions can also be used. Usually the energy of electrons and photons is comprised between 1 MeV and several MeV. The energy of protons may sometimes be as high as 240 MeV. The dose delivered is usually comprised between 1 and 100 Gy.

This phantom allows to measure simultaneously the dose with both radiation sensitive film (measuring a relative dose) and at least one radiation detector measuring an absolute dose. The absolute dose is then used to quickly calibrate the film.

Preferably, the radiation sensitive film is a radiographic or radiochromic film. More preferably, the radiation sensitive film is a radiochromic film.

Preferably, a cross section of the receptacle is curved with one or more portions selected from at least one of: a spiral, a parabola, a hyperbola, a circle, an ellipse, a polynomial expression, a logarithm curve, an exponential curve.
This non-plane shape of the receptacle allows to have a very high spatial resolution in all the direction. For example, in radiotherapy, the beam source is mounted on a gantry and its orientation may change. It is therefore crucial to correctly measure de received dose for any orientation of the gantry. Preferably, the film should have portions perpendicular or nearly perpendicular to all possible orientation of the beam to ensure a good measurement. An area of the film where the beam impinges tangentially is not usable.

Preferably, the receptacle has a width not more than the thickness of the radiation sensitive film plus one millimetre. More preferably, the receptacle is as thin as possible in order to reduce the interaction of a radiation with the air of the thin air gap between the film and the receptacle. This air gap can perturb the measurements.

In an embodiment, the main body of material of the phantom comprises at least two receptacles respectively adapted for inserting a radiation sensitive film each. In this configuration, one film can be used for measuring the dose form a radiation beam emitted with an orientation equal to 0 to 180° and the second film is used form the measurement with the orientation between 180° and 360°. One can therefore cover all possible orientations.

Preferentially, the one or more radiation detectors comprise ionization chamber detectors and/or diode detectors. These ionization chamber detectors measure precisely dose in region of uniform dose. Diode detectors can characterize steep dose gradients. Both detectors are able to measure absolute dose but their spatial resolution is less good than radiation sensitive film.

Preferentially, the main body of material comprises a plurality of radiation detectors arranged in an array, preferably, the array is a linear array or a 2D array. More preferentially, the radiation sensitive film has a length and a width and the array of detectors extends within the length or the width of the radiation sensitive film.
This configuration allows to measure the absolute dose in several key point such the point receiving the maximum dose, the point receiving the minimum dose and some intermediate points. These points are very useful for the calibration of the film.

Preferably, the at least one radiation detector is in contact with a portion of the radiation sensitive film. This facilitates the search of the corresponding region between the absolute dose received by the detector and the relative dose received by the film as these regions are in contact.

Preferably, the receptacle comprises an opening at at least one external surface of the main body of material, preferably the receptacle is a slot. This characteristic allows to drop or insert the film precisely and correctly.

Preferentially, the main body of material comprises at least two blocks of material, at least one block of material having a surface having a shape matching the shape of the surface comprising a receptacle of another block of material.

Preferably, the phantom furthermore comprises a film identifier reader, and the radiation sensitive film comprises an identifier. This allows to directly identify the absolute measure of the detectors with the right film.

According to a second aspect, the invention refers to a method for calibrating a radiation sensitive film. The first steps of this method comprises the providing of a phantom, a radiation source, a radiation sensitive film scanner, and a controller. These steps consist in providing: a phantom comprising a main body of material comprising a receptacle having the shape of a developable surface, and a radiation sensitive film located in the said receptacle, the said main body of material further comprises one or more radiation detectors each for respectively measuring a dose of radiation which reached a specific location of said radiation sensitive film; a radiation source able to emit a radiation beam in at least one direction; a radiation sensitive film scanner; a controller.
The next step consists in placing the phantom such that the radiation beam passes through the radiation sensitive film and through the one or more radiation detectors. Then one emits a radiation beam and one measures at least one part of the radiation simultaneously with the radiation sensitive film and with the at least one or more radiation detectors.
Then the film is scanned and the dose relative distribution corresponding to absolute doses measured with at least one of the one or more radiation detectors is extracted from the film image. A calibration curve is computed by comparing the relative dose distributions of the said scanned film 2D image to the absolute doses of the one or more radiation detectors. Finally, the calibration curve is applied to all the points of scanned film image and the scanned film image is converted in dose units.
The controller can scan the film, compute and apply the curve automatically. This method allows to calibrate quickly and reliably the radiation sensitive film thanks to the absolute measurement of the detectors.

Preferentially, the phantom used is a phantom such as described above.

Preferentially, the phantom provided comprises an identifier reader and the film comprises an identifier. The method further comprises the step of reading of the film identifier with the film identifier reader of the phantom. This step is executed before measuring the radiation. This step can be made automatically under control of the controller.

According to a third aspect, the invention relates to a device for radiation dosimetry comprising a phantom as described above, and a controller configured for executing several steps of the method as described above.

### Short description of the drawings

These and further aspects of the invention will be explained in greater detail by way of example and with reference to the accompanying drawings in which:
Fig.1 schematically shows a first embodiment of a phantom for radiation dosimetry according to the invention;
Fig.1a shows examples of developpable surfaces;
Fig.1b shows examples of cross section of receptacle;
Fig 1c shows an example of the first embodiment comprising a reader and inserts and a radiation sensitive film;
Fig.1d shows examples of radiation detectors organisations;
Fig.1e shows examples of relative positions of the radiation sensitive film with respect to a radiation detector.
Fig.2a schematically shows a first example of a second embodiment of a phantom for radiation dosimetry according to the invention;
Fig.2b schematically shows a second example of a second embodiment of a phantom for radiation dosimetry according to the invention;
Fig.3 shows a more complex example of the second embodiment of a phantom according to the invention;
Fig.4 shows a third embodiment of a phantom according to the invention.;
Fig.4a schematically shows an example of the third embodiment comprising two slots;
Fig.5a shows a diagram of an example of the calibration method for a radiation sensitive film;
Fig.5b shows an example of calibration curves.

The figures are not drawn to scale. Generally, identical components are denoted by the same reference numerals in the figures.

### Detailed description of preferred embodiments

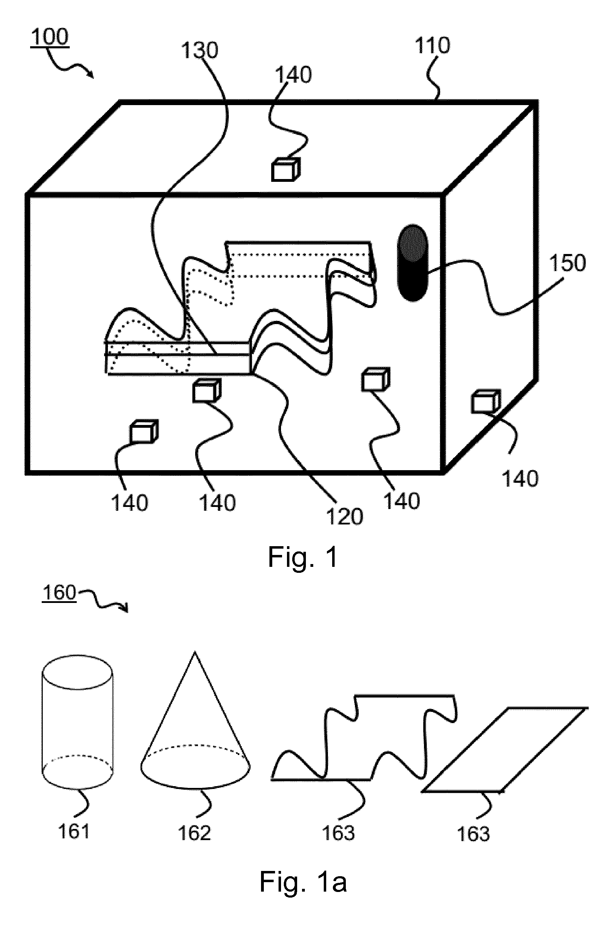

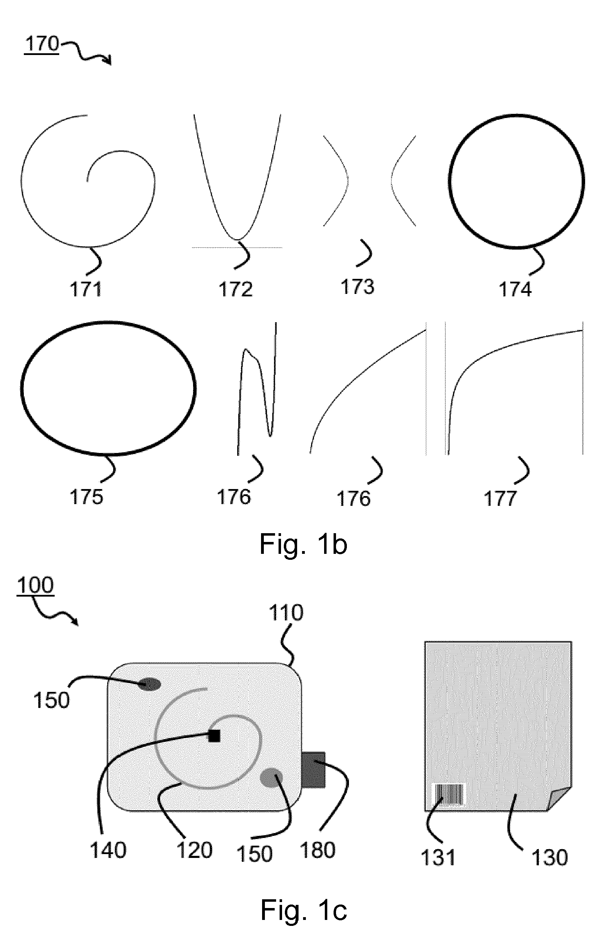

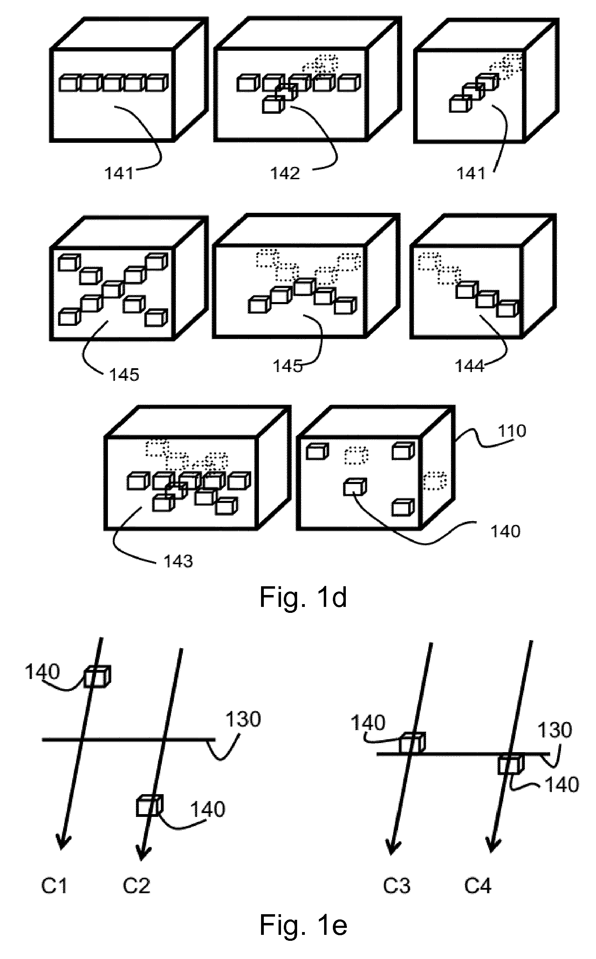

Figure 1 shows a first embodiment of a phantom 100 for radiation dosimetry according to the invention. The phantom 100 comprises a main body of material 110 comprising a receptacle 120 and a radiation sensitive film 130 located in the said receptacle 120. The phantom 100 also comprises one or more radiation detectors 140 each for measuring a dose of radiation which reached a specific location of said radiation sensitive film 130.

For example, the main body of material 110 can be made of plastic, or resin-based solid substitute for water. Preferentially, the main body of material 110 is a rigid body. The main body of material 110 has to be sufficiently transparent to the radiation measured by the detector 140 and film 130. The main body of material 110 can have any shape. For instance, it has the shape of a prism. It can also have the shape of a human body. The main body of material 110 can comprise one or more inserts 150. These inserts 150 have different densities and are introduced to mimic particular features of human body, for example: high density bones, low density lungs, air cavity in rectum.

Preferentially, the receptacle 120 has the shape of a developable surface 160. Some examples of developable surfaces 160 are shown in Fig 1 a. A developable surface 160 is a surface that can be flattened onto a plane without distortion. For example, the surface can be a cylinder 161, a cone 162 or a conical surface, an oloid or a plane 163. Preferably, the receptacle 120 has a minimal size equal to the size of the radiation sensitive film 130. For example, the EBT2 films manufactured by Ashland Inc. (http://www.ashland.com) have a size of 43cm (or 25cm) of length to 32cm (or 20cm) of width. For these films, the receptacle 120 should have a minimal size of 43cm (or 25cm) of length to 32cm (or 20cm) of width in order to host a film 130.

Preferentially, the receptacle 120 presents a cross section which is a portion of at least one of the following curve 170 (see Fig 1b): a spiral 171, a parabola 172, a hyperbola 173, a circle 174, an ellipse 175, a polynomial expression 176, a logarithm curve 177, an exponential curve.

The shape of the receptacle 120 is crucial to properly cover the area that has to be measured. For example, during the pre-treatment or online plan verification, it is important to precisely measure the dose distribution in the area that will be irradiated during the treatment in order to reduce the destruction of healthy cells.

The radiation sensitive films 130 have a length, a width and a thickness. Preferably, the receptacle 120 has a minimum depth equal to the length or to the width of the film 130, a minimum width equal to the width or to the length of the film 130 and a maximum width equal to the thickness of the plus one millimetre. For example, the EBT2 films have a thickness of 0.29mm, in this case, the maximal width of the receptacle 120 is 1,29mm. More preferably, the receptacle 120 is thin as possible in order to reduce the interaction of a radiation with the air of the thin air gap between the film 130 and the receptacle 120.

Preferentially, the main body of material 110 of the phantom 100 for radiation dosimetry comprises at least two receptacles 120 respectively adapted for receiving a radiation sensitive film 130 each.

Preferably, the radiation sensitive film 130 comprises an identifier 131 (see Fig 1c), for example: a bar code, a QR code, a chip, an ID TAG. This identifier 131 can be located in a corner of the film 130 and can serve to know the relative orientation of the film 130 with respect to the receptacle 120.

Preferentially, the phantom 100 comprises a film identifier reader 180 to read the film identifier 131. This reader 180 can be a bar code reader, a QR code reader, an ID TAG reader, or an electronic card reader.

Preferentially, the one or more radiation detectors 140 comprise ionization chamber detectors, diode detectors, or a combination of the two. These two types of detector 140 have both their advantages and inconvenient. For instance, ionization chamber detectors can measure precisely dose in region of uniform dose, they are very stable and exhibit a very week sensitivity dependence on beam quality. Diode detectors can characterize steep dose gradients; they can also be easily produced with sub-mm size.

The most important difference between these detectors 140 and sensitive films 130 is that they are connected to a readout electronics, and that the measurement is automatically available at the end of the irradiation, without further actions or waiting time. However, ionization chamber detectors cannot be too small for manufacturing reasons and because one needs a minimum sensitivity to have a good signal to noise ratio (at least few mm³ of air volume). The diode detectors suffer from sensitivity changes with delivered dose, dose rate and beam quality.

Preferentially, the one or more radiation detectors 140 have a density and/or a chemical composition which is close to the density and/or a chemical composition of the main body of material 110.

Preferentially, the main body of material 110 comprises a plurality of radiation detectors 140 located in an array. More preferentially, this array is a linear array or a 2D array. Fig 1d shows some examples of array organisations. The detectors 140 can be arranged in various shapes. For example, they can form a line 141, a cross 142, a star 143, a diagonal 144, two diagonals 145. They preferentially lay in the same plane. They are preferentially disposed along the shape of the receptacle 120. Alternatively, they can lay in a plurality of planes.

The detectors 140 can be included in the main body of material 110 during its fabrication. Preferably, the main body of material 110 comprises slots to insert the detectors 140 or the array of detectors 140. In this way, the detectors 140 can be removed in case of problems or be used in another phantom 100. More preferably, the slots for the detectors 140 are in contact with the film receptacle 120.

Preferentially, at least a portion the radiation sensitive film 130 is in contact with at least one radiation detector 140. More preferentially all the radiation detectors 140 are in contact with the radiation sensitive film 130.

Preferentially, the radiation sensitive film 130 has a length and a width and the array extends through the width or the length of the radiation sensitive film 130.

Figure 1e shows examples of relative positions of the radiation sensitive film 130 with respect to a radiation detector 140. For example, a radiation beam 190 can firstly pass through a radiation detector 140 and then through a radiation sensitive film 130 (case C1). In this example, the radiation detector 140 may be transparent or nearly transparent to the radiation beam 190. Alternatively, the radiation detector 140 may have an absorption coefficient which is equal or nearly equal to the absorption coefficient of the surrounding medium (i.e. the main body of material 110). The detector 140 is preferably in contact with the film 130 (case C3). In another example, a radiation beam 190 can firstly pass through a radiation sensitive film 130 and then through a radiation detector 140 (case C2). In this example, the radiation sensitive film 130 may be transparent or nearly transparent to the radiation beam 190. Alternatively, the radiation sensitive film 130 may have an absorption coefficient which is equal or nearly equal to the absorption coefficient of the surrounding medium (i.e. the main body of material 110). The detector 140 is preferably in contact with the film 130 (case C4).

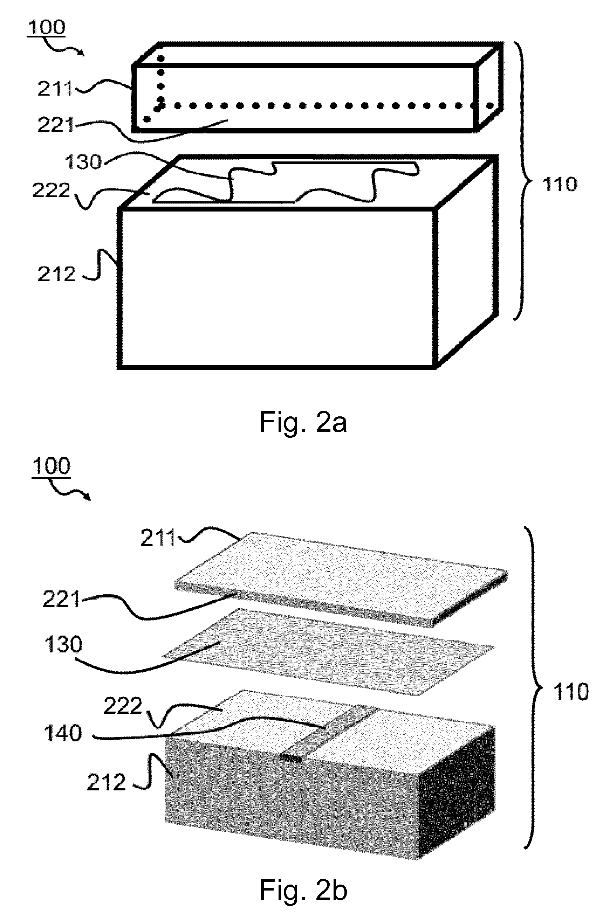

Figure 2a and 2b shows an example of another embodiment of a phantom 100 according to the invention. In this embodiment, the receptacle 120 is at an external surface 222 of the main body of material 110. Thus the receptacle 120 is open and the film 130 has to be dropped into the receptacle 120. Again, the receptacle 120 has the shape of a developable surface 160. Preferentially, the main body of material 110 of the phantom 100 is made of two blocks of material 211, 212. The first block 211 has an external surface 221 that matches the shape of the external surface 222 of the second block 212 that comprises the receptacle 120, in order to perfectly cover said second block 212.

The one or more radiation detectors 140 can be comprised in the first 211 and/or in the second block of material 212 of the main body of material 110. Preferentially, the one or more radiation detectors 140 are in the same block as the receptacle 120. More preferentially, they are in contact with the receptacle 120 (see Fig 2a).

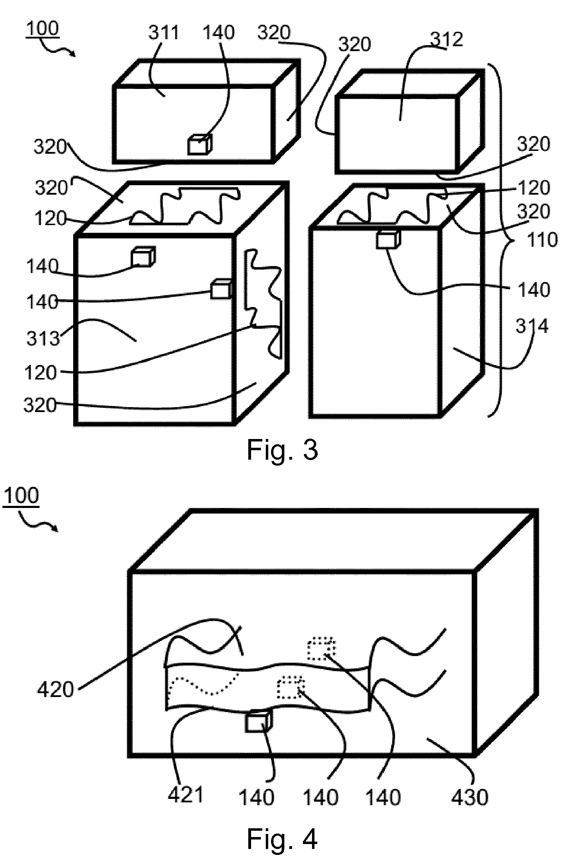

Figure 3 shows a more complex example of the second embodiment of a phantom 100 according to the invention. In this embodiment, the main body of material 110 is made of a plurality of blocks 311, 312, 313, 314. These blocks 311, 312, 313, 314 have at least one external surface 320 which can be in contact with an external surface 320 of at least one other block 311, 312, 313, 314. These surfaces 320 are called inside surfaces. Preferentially, these inside surfaces 320 have a shape matching the shape of the surface 320 of the said at least one other block 311, 312, 313, 314 with which they are in contact.

Preferentially, blocks 311, 312, 313, 314 that have at least two inside surfaces 320 can optionally comprises at least one additional receptacle 120 located on an inside surface 320 and comprising an opening. An additional radiation sensitive film 130 is located in the said additional receptacle 120.

Preferentially, the one or more radiation detectors 140 can be comprised in several blocks (for example, 311, 313, 314) of material of the main body of material 110. Preferentially, the radiation detectors 140 are in the same block(s) as the receptacle(s) 120. More preferentially, they are in contact with the receptacle(s) 120.

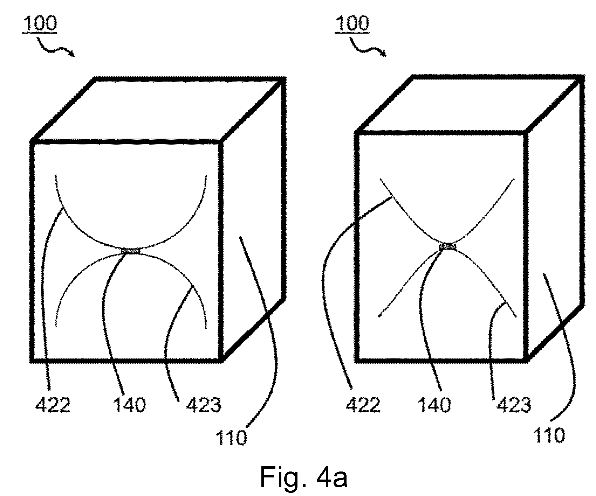

Figure 4 shows a third embodiment of a phantom 100 according to the invention. In this embodiment, the receptacle 120 comprised in the main body of material 110 is a slot 420 comprising an opening 421 at at least one external surface 430 of the main body of material 110 and adapted for inserting a radiation sensitive film 130.

Preferentially, the slot 420 presents a cross section which is a portion of at least one of the following curve 170: a spiral 171, a parabola 172, a hyperbola 173, a circle 174, an ellipse 175, a polynomial expression 176, a logarithm curve 177, an exponential curve.

The radiation sensitive films 130 have a length, a width and a thickness. Preferably, the slot 420 has a minimum depth equal to the length or to the width of the film 130, a minimum width equal to the width or to the length of the film 130 and a maximum width equal to the thickness of the plus one millimetre. For example, the EBT2 films have a thickness of 0.29mm, in this case, the maximal width of the slot 420 is 1,29mm. More preferably, the slot 420 is thin as possible in order to reduce the interaction of a radiation with the air of the thin air gap between the film 130 and the slot 420.

Preferentially, the main body of material 110 of the phantom 100 for radiation dosimetry comprises at least two slots 422, 423 respectively adapted for inserting a radiation sensitive film 130 each. Figure 4b shows two examples of configuration of two slots 422, 423. These slots 422, 423 have the same characteristics as the one explained above for one slot 420.

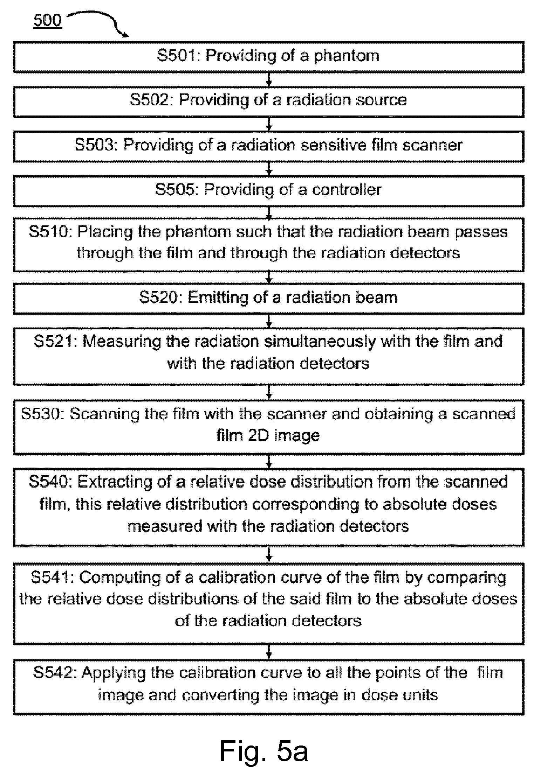

According to a second aspect, the invention relates to a method 500 for calibrating a radiation sensitive film 130. Figure 5a shows an example of diagram of the method 500. This method 500 comprises the provision of a phantom 100 such as described above S501.

The next step comprises the provision of a radiation source being able to emit a radiation beam 190 in at least one direction S502. The radiation source may be part of a radiotherapy machine comprising an accelerator, or may comprise a radioactive material. One also provides a dosimetry sensitive film scanner S503.

The entire phantom 100 is then placed under the radiation beam 190 path S510 such that the radiation beam 190 passes through the radiation sensitive film 130 and through the one or more radiation detectors 140. Preferably, at least a portion of the film 130 and one or more radiation detectors 140 are located at the isocenter. The isocenter is, in a radiotherapy machine, the point relative to the treatment machine around which various components of the linear accelerator rotate. Usually, the maximum dose is delivered at isocenter. During a treatment procedure, the centre of a target volume is preferably placed at or near the isocenter of the treatment unit.

Preferentially, the relative orientation of the film 130 is automatically recorded. For example, one may add one or few needles to the cover body, in order to stamp the film 130 at given positions along its edge. Alternatively, one could add a piece of metal above a given position along one edge of the film 130. Another possibility would be the use of a snick on one edge of the film 130.

Preferentially, the film 130 also comprises a marker for its identification. For example: a bar code, a QR code, a chip, an IDTAG. This identifier 131 can be located in a corner of the film 130 and can also serve to know the relative orientation of the film 130 with respect to the receptacle 120. This identifier 131 can be scanned by an identifier reader 180 of the phantom 100 before radiation measurement in order to associate a film 130 to detectors 140 data.

A radiation beam 190 delivering a radiation is emitted S520 and at least a part of the radiation is measured simultaneously S521 with the radiation sensitive film 130 and with the one or more radiation detectors 140. Once the irradiation is ended, the film 130 is removed from the receptacle 120 and scanned with the scanner S530. Preferentially, the identifier 131 of the film 130 is visible on the scan.

Preferentially, the radiation is measured by the film 130 and the detector 140 at the isocenter or near the isocenter. This measurement point corresponds to the highest dose used in the treatment, and the measurement of this dose increases the quality of calibration. Preferentially, the detectors 140 extend from the isocenter to one edge of the film 130. At the edge, the dose measured is very low and close to zero. This measurement point gives a calibration point that is close to the origin. These points allow to improve the quality of the calibration.

Preferentially, an array of detectors 140 is used, which is provide many points in the full dose range delivered to the patient. This allow to improve the quality of the calibration curve. More preferentially, more than a simple linear array (e.g. 2 arrays of a 2D array) introduces a redundancy that helps to deal with plan variability and improve again the quality of the calibration curve. This also allows to account for the cases where the highest dose is not at isocenter and/or for the cases where the dose gradient along on line may be very.

The scanned 2D image of the film 130 is treated to extract a relative dose distribution S540 corresponding to absolute dose(s) that have been measured with the one or more radiation detectors 140.

The relative dose distribution extracted from the film 2D image is compared to the absolute dose(s) S541 of the one or more radiation detectors 140 to compute a calibration curve.

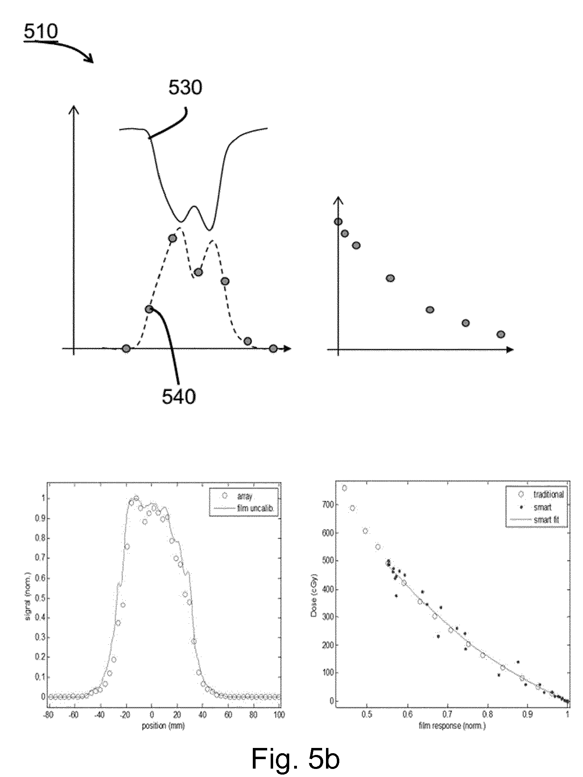

Preferentially, the dose distribution extracted from the film image is divided by the dose distribution measured with the one or more radiation detectors 140. In the case of one radiation detector 140, we get one point. If the shape of the calibration curve is known, which is sometimes the case, one can fit a calibration curve of the radiation sensitive film 130 passing through this point. Preferentially, one has more than one detector 140 and one has several points through which a calibration curve can be fitted. The fitting procedure can be a polynomial, linear, quadratic, or logarithmic fitting. Preferably, it is a smooth fitting procedure or a least square minimisation. Figure 5b shows an example of calibration curves. Figure 5b left shows the relative dose distribution extracted from the film 2D image 530 and the absolute doses 540 that have been measured with the one or more radiation detectors 140 (top: hypothetic case, bottom experimental results). Figure 5b top right shows the points that the calibration curve has to fit. Figure 5b bottom right shows the points that the calibration curve has to fit (smart) as well as a calibration curve (smart fit) obtained for experimental results compared to the traditional film calibrating method (traditional).

Finally, the calibration curve S542 is applied to all the points of the radiation sensitive film 2D image and the image is converted in dose units.

Preferentially, the method for calibrating a radiation sensitive film 130 comprises the provision of a controller. This controller is configured to automatically compute the calibration curve of the radiation sensitive film 130.

Preferentially, the controller is configured to automatically associate a film scan to detector 140 data thanks to the film identifier 131. This identifier 131 can be automatically read by the phantom 100 before the radiation measurement.

Preferentially, the device according to the invention comprises a phantom 100 as described above, and a controller configured for executing the steps described above.

The present invention has been described in terms of specific embodiments, which are illustrative of the invention and not to be construed as limiting. More generally, it will be appreciated by persons skilled in the art that the present invention is not limited by what has been particularly shown and/or described hereinabove. The invention resides in each and every novel characteristic feature and each and every combination of characteristic features. Reference numerals in the claims do not limit their protective scope. Use of the verbs "to comprise", "to include", "to be composed of", or any other variant, as well as their respective conjugations, does not exclude the presence of elements other than those stated. Use of the article "a", "an" or "the" preceding an element does not exclude the presence of a plurality of such elements.

Summarized, the invention may also be described as follows. Phantom for radiation dosimetry comprising a main body of material, a receptacle having the shape of a developable surface, a radiation sensitive film located in the said receptacle, the main body of material comprises one or more radiation detectors each for measuring a dose received at a location of said radiation sensitive film. The invention also relates to the method for calibrating a radiation sensitive film used in the phantom according to the invention.

## Claims

1. Phantom (100) for radiation dosimetry comprising a main body of material (110) comprising a receptacle (120) having the shape of a developable surface (160), and a radiation sensitive film (130) located in the said receptacle (120),
**characterised in that**,
said main body of material (110) further comprises one or more radiation detectors (140) each for respectively measuring a dose of radiation which reached a specific location of said radiation sensitive film (130).

2. Phantom (100) for radiation dosimetry according to claim 1, wherein a cross section of the receptacle (120) is curved with one or more selected from at least one of: a spiral (171), a parabola (172), a hyperbola (173), a circle (174), an ellipse (175), a polynomial expression (176), a logarithm curve (177), an exponential curve.

3. Phantom (100) according to anyone of the previous claims, wherein the receptacle (120) has a width not more than the thickness of the radiation sensitive film (130) plus one millimetre.

4. Phantom (100) for radiation dosimetry according to anyone of the previous claims, wherein the main body of material (110) comprises at least two receptacles (120) respectively adapted for inserting a radiation sensitive film (130) each.

5. Phantom (100) for radiation dosimetry according to any of the previous claims, wherein the one or more radiation detectors (140) comprise ionization chamber detectors and/or diode detectors.

6. Phantom (100) for radiation dosimetry according to any of the previous claims, wherein it comprises a plurality of radiation detectors (140) arranged in an array, preferably, the array is a linear array or a 2D array.

7. Phantom (100) for radiation dosimetry according to any of the previous claims, wherein the radiation sensitive film (130) has a length and a width and **characterised in that** array extends within the length or the width of the radiation sensitive film (130).

8. Phantom (100) for radiation dosimetry according to any of the previous claims, wherein at least one radiation detector (140) is in contact with a portion of the radiation sensitive film (130).

9. Phantom (100) for radiation dosimetry according to any of the previous claims, wherein said receptacle (120) comprises an opening (421) at at least one external surface (222, 320, 430) of the main body of material (110), preferably the receptacle (120) is a slot (420, 422, 423).

10. Phantom (100) for radiation dosimetry according to claim 9, wherein said main body of material (110) comprises at least two blocks of material (211, 212, 311, 312, 313, 314), at least one block of material (211, 212, 311, 312, 313, 314) having a surface (221, 320, 430) having a shape matching the shape of the surface (222, 320, 430) comprising a receptacle (120) of another block of material (211, 212, 311, 312, 313, 314).

11. Phantom (100) for radiation dosimetry according to any of the previous claims, wherein said phantom (100) furthermore comprises a film identifier reader (180), and wherein said radiation sensitive film (130) comprises an identifier (131).

12. Method for calibrating a radiation sensitive film (130) comprising the steps:
a. providing of a phantom (100) comprising a main body of material (110) comprising a receptacle (120) having the shape of a developable surface (160), and a radiation sensitive film (130) located in the said receptacle (120), the said main body of material (110) further comprises one or more radiation detectors (140) each for respectively measuring a dose of radiation which reached a specific location of said radiation sensitive film (130) (S501);
b. providing of a radiation source able to emit a radiation beam (190) in at least one direction (S502);
c. providing of a radiation sensitive film scanner (S503);
d. providing of a controller (S504);
e. placing the phantom (100) such that the radiation beam (190) passes through the radiation sensitive film (130) and through the one or more radiation detectors (140) (S510);
f. emitting of a radiation beam (190) (S520);
g. measuring at least one part of the radiation simultaneously with the radiation sensitive film (130) and with the at least one or more radiation detectors (140) (S521);
h. scanning the radiation sensitive film (130) with the scanner and obtaining a scanned film 2D image (S530);
i. extracting of a relative dose distribution from the scanned film 2D image, this relative distribution corresponding to absolute doses measured with at least one of the one or more radiation detectors (140) (S540);
j. computing of a calibration curve of the scanned film 2D image by comparing the relative dose distributions of the said scanned film 2D image to the absolute doses of the one or more radiation detectors (140) (S541);
k. applying the calibration curve to all the points of scanned film 2D image and converting the scanned film 2D image in dose units (S542);
the step d is optional and at least one of the steps f, h to j can be made automatically under control of the controller.

13. Method for calibrating a radiation sensitive film (130) according to claim 12, wherein the phantom provided in the step a is a phantom (100) according to any of the claims 2 to 11.

14. Method for calibrating a radiation sensitive film (130) according to claim 13, wherein the phantom (100) provided in step a. is a phantom (100) according to claim 11 and wherein the method further comprises the step: reading of the film identifier (131) with the film identifier reader (180) of the phantom (100); this step being executed before step g. of claim 12, this step can be made automatically under control of the controller.

15. Device for radiation dosimetry comprising a phantom (100) according to any of the claims 1 to 11, and a controller configured for executing the steps f, h to j of claim 12.

16. Device for radiation dosimetry according to claim 14, wherein the phantom (100) is a phantom (100) according to claim 11, and wherein the controller is further configured for executing the additional step of claim 13.
